# EUROPEAN PATENT SPECIFICATION

(11) **EP 4 403 926 B1**
(45) Date of publication and mention of the grant of the patent: **29.04.2026**
(21) Application number: 23212941.1
(22) Date of filing: 29.11.2023
(51) Int. Cl.: G01N 35/10, G01N 35/00

(54) **CONSUMABLE RECYCLING APPARATUS AND NUCLEIC ACID TEST ANALYZER**
VERBRAUCHBARE RECYCLINGVORRICHTUNG UND NUKLEINSÄURETESTANALYSEGERÄT
APPAREIL DE RECYCLAGE DE PRODUITS CONSOMMABLES ET ANALYSEUR D'ESSAI D'ACIDE NUCLÉIQUE

(30) Priority: 19.01.2023 CN 202310101119
(43) Date of publication of application: 24.07.2024
(73) Proprietor: Shenzhen New Industries Biomedical Engineering Co., Ltd., Shenzhen, Guangdong 518122 (CN)
(72) Inventor: XIAO, Hao, Shenzhen, 518122 (CN); HE, Guoyao, Shenzhen, 518122 (CN); XIONG, Yingchao, Shenzhen, 518122 (CN); QIU, Zefeng, Shenzhen, 518122 (CN); PENG, Yangyang, Shenzhen, 518122 (CN); SHI, Hao, Shenzhen, 518122 (CN); ZHANG, Yuan, Shenzhen, 518122 (CN)
(74) Representative: Studio Torta S.p.A.

(56) References cited:
- WO-A1-2015/062548
- WO-A2-2014/022532
- CN-A- 111 948 415
- CN-A- 114 441 510

## Description

### Technical Field

The present invention relates to the field of nucleic acid tests, and particularly relates to a consumable recycling apparatus and a nucleic acid test analyzer.

### Background

Consumables are frequently used in a nucleic acid test analyzer so as to complete nucleic acid extraction. Specifically, the consumables include a nucleic acid extraction plate, a magnetic rod sleeve, etc. For the nucleic acid test analyzer, a consumable recycling apparatus needs to be arranged to recycle the nucleic acid extraction plate, the magnetic rod sleeve, etc. Meanwhile, liquid in the extraction plate needs to be recycled.

In the related art, the consumable recycling apparatus includes an extraction plate recycling apparatus, a magnetic rod sleeve recycling apparatus and a liquid recycling apparatus. Being arranged separately, these apparatuses occupy too much space in the nucleic acid test analyzer, resulting in that an overall space utilization rate of the analyzer is low.

Examples of System, Method, and Apparatus for Automated Incubation according to the prior art are known from WO2014022532A2.

### Summary

A main objective of the present invention is to provide a consumable recycling apparatus and a nucleic acid test analyzer, so as to solve a problem that a consumable recycling apparatus in the related art occupies a large space.

The invention is set out in the appended set of claims.

In order to achieve the above objective, according to one aspect of the present invention, a consumable recycling apparatus is provided. The consumable recycling apparatus includes: a bearing frame; a supporting plate structure arranged on the bearing frame, wherein the supporting plate structure is provided with a consumable bearing area; a recycling structure adjacent to the bearing frame, wherein the recycling structure includes a rack, a mounting beam movably arranged on the rack, and a driving structure arranged on the rack and driving the mounting beam to move, the mounting beam is provided with a plurality of recycling rods arranged at intervals in a horizontal direction, and each of the recycling rods is internally provided with an airflow channel; and a stripping structure arranged on the bearing frame and spaced from the supporting plate structure, wherein the stripping structure includes a plurality of notch structures, the plurality of notch structures are arranged in one-to-one correspondence with the plurality of recycling rods, and a width of the notch structure is greater than a diameter of the recycling rod, wherein the mounting beam has a working position for cooperating with the supporting plate structure and a separating position for cooperating with the stripping structure, when the mounting beam is at the working position, the recycling rod is capable of moving close to or away from the supporting plate structure, and when the mounting beam is at the separating position, each of the recycling rods extends into one of the notch structures.

In some embodiments, the stripping structure and the supporting plate structure are arranged at an interval in a preset direction , an opening of the notch structure faces the supporting plate structure, the rack includes a fixed plate and a movable plate movably arranged on the fixed plate in the preset direction , the mounting beam is movably arranged on the movable plate in a vertical direction, the driving structure includes a horizontal driving structure and a vertical driving structure, the horizontal driving structure is arranged between the fixed plate and the movable plate so as to drive the movable plate to move, and the vertical driving structure is arranged between the movable plate and the mounting beam so as to drive the mounting beam to move.

In some embodiments, the bearing frame includes a guide housing and a vertical plate arranged at a side of the guide housing, the guide housing is internally provided with a recycling channel, the stripping structure is arranged at an entrance of the recycling channel, and the supporting plate structure is arranged on the vertical plate.

In some embodiments, the supporting plate structure is movably arranged on the bearing frame in the vertical direction, and the consumable recycling apparatus further includes: a separating frame arranged on the bearing frame, wherein the separating frame is provided with an avoidance hole for the supporting plate structure to pass downward.

In some embodiments, the consumable recycling apparatus further includes: a stop structure movably arranged on the vertical plate, wherein the stop structure has a stop position below the supporting plate structure and an avoidance position misaligned with the supporting plate structure, and when the mounting beam is at the working position, the stop structure is at the stop position so as to support the supporting plate structure.

In some embodiments, the stop structure includes a driving motor and a swinging member arranged on the vertical plate, a first end of the swinging member is in driving connection with a pivot shaft of the driving motor, a second end of the swinging member has the stop position and the avoidance position, and the driving motor is capable of driving the second end of the swinging member to move between the stop position and the avoidance position.

In some embodiments, the driving motor penetrates the vertical plate, the vertical plate is further provided with an arc-shaped guide groove, and the second end of the swinging member is provided with a supporting rod capable of sliding in the arc-shaped guide groove.

In some embodiments, the consumable recycling apparatus further includes: a pushing structure including a mounting seat arranged on the bearing frame and a push rod movably arranged on the mounting seat, wherein the push rod is capable of pushing a consumable to leave the separating frame.

In some embodiments, the supporting plate structure is provided with a limiting structure configured to limit the consumable.

In some embodiments, the limiting structure includes a plurality of limiting columns arranged on the supporting plate structure; and/or the limiting structure includes a magnetic attraction structure arranged on the supporting plate structure.

In some embodiments, the recycling rod includes a first rod section connected to the mounting beam and a second rod section located below the first rod section, a diameter of the first rod section is greater than a diameter of the second rod section, the first rod section is provided with a diameter-changing portion having a diameter gradually decreasing from top to bottom, and the airflow channel penetrates the first rod section and the second rod section.

According to another aspect of the present invention, a nucleic acid test analyzer is provided, which includes a consumable recycling apparatus. The consumable recycling apparatus is the consumable recycling apparatus described above.

By applying the technical solution of the present invention, the consumable recycling apparatus includes the bearing frame, the bearing frame is provided with the supporting plate structure, the supporting plate structure is provided with the consumable bearing area, and a nucleic acid extraction plate may be placed on the consumable bearing area. The consumable recycling apparatus further includes the recycling structure and the stripping structure. The recycling structure includes the rack, the mounting beam movably arranged on the rack, and the driving structure arranged on the rack and driving the mounting beam to move. The mounting beam is provided with the plurality of recycling rods arranged at intervals in an extension direction of the mounting beam. The recycling rod may be driven by the driving structure to penetrate into a magnetic rod sleeve, such that the recycling rod may be inserted into the magnetic rod sleeve. Then the driving structure may move the recycling rod having the magnetic rod sleeve into the notch structure on the stripping structure, and enable the magnetic rod sleeve to be located below the stripping structure. The width of the notch structure is greater than the diameter of the recycling rod, such that the recycling rod may be driven by the driving structure to move upward, the magnetic rod sleeve having a greater diameter may be stopped by the stripping structure and stripped, and the recycling rod may be pulled out from the notch structure, so as to achieve the stripping of the magnetic rod sleeve. Meanwhile, the recycling rod is further internally provided with the airflow channel, and the airflow channel is in communication with a negative pressure device, such that the recycling rod may further extend into an accommodating hole on the nucleic acid extraction plate so as to suck liquid from the nucleic acid extraction plate, and the waste liquid is recycled. By designing the integrated consumable recycling apparatus, the above structure reduces space occupied by the consumable recycling apparatus and improves a space utilization rate of the nucleic acid test analyzer.

### Brief Description of the Drawings

The drawings of the description as a constituent part of the invention are used to provide further understanding of the present invention, and illustrative embodiments of the present invention and the description thereof are used to explain the present invention, which are not intended to unduly limit the present invention. In the drawings:
Fig. 1 shows a schematic diagram of a three-dimensional structure of an embodiment of a consumable recycling apparatus according to the present invention;
Fig. 2 shows a schematic diagram of a three-dimensional structure of a partial structure of the consumable recycling apparatus in Fig. 1;
Fig. 3 shows a schematic diagram of a three-dimensional structure of a vertical plate of the consumable recycling apparatus in Fig. 1;
Fig. 4 shows a schematic diagram of an enlarged structure of part A of the vertical plate in Fig. 3;
Fig. 5 shows a schematic structural diagram of the consumable recycling apparatus in Fig. 1 when recycling rods cooperate with magnetic rod sleeves;
Fig. 6 shows a schematic structural diagram of a recycling rod of the consumable recycling apparatus in Fig. 1.

The above figures involve the following reference numerals:
10, bearing frame; 11, guide housing; 12, vertical plate; 121, arc-shaped guide groove; 20, supporting plate structure;
30, recycling structure; 31, rack; 311, fixed plate; 312, movable plate; 32, mounting beam; 33, driving structure; 331, horizontal driving structure; 332, vertical driving structure; 34, recycling rod; 341, first rod section; 342, second rod section; 3421, notch;
40, stripping structure; 41, notch structure;
50, separating frame;
60, stop structure; 61, driving motor; 62, swinging member; 621, supporting rod;
71, limiting column; 72, magnetic attraction structure;
80, pushing structure;
90, magnetic rod sleeve; 100, nucleic acid extraction plate.

### Detailed Description of the Embodiments

Technical solutions of embodiments of the present invention are clearly and completely described below with reference to the accompanying drawings in the embodiments of the present invention. Obviously, the described embodiments are merely some embodiments rather than all embodiments of the present invention. The following description of at least one illustrative embodiment is merely illustrative in nature and in no way serves as any limitation of the present invention and its application or uses. Based on the embodiments of the present invention, all the other embodiments obtained by those of ordinary skill in the art without inventive effort fall within the protection scope of the present invention.

It should be noted that the terms used herein are merely for describing the detailed description of embodiments and are not intended to limit illustrative embodiments according to the invention. As used herein, the singular is also intended to include the plural unless the context clearly indicates the singular or plural. In addition, it should be understood that terms "include" and/or "comprise" used in the description indicate the presence of features, steps, operations, devices, assemblies, and/or their combinations.

It should be noted that the relative arrangement, numerical expressions and numerical values of components and steps described in the embodiments do not limit the scope of the present invention unless otherwise specified. Moreover, it should be understood that for convenience of description, dimensions of each part shown in the drawings are not drawn according to a real proportional relation. Technologies, methods and devices known to those of ordinary skill in related fields may not be discussed in detail, but in appropriate cases, they should be regarded as part of the description. In all the instances shown and discussed herein, any specific value should be interpreted as exemplary only, not as a limitation. Therefore, other instances of the illustrative embodiments may have different values. It should be noted that like numerals and letters denote like items in the following accompanying drawings, and therefore, once an item is defined in one accompanying drawing, it does not need to be further discussed in the subsequent accompanying drawings.

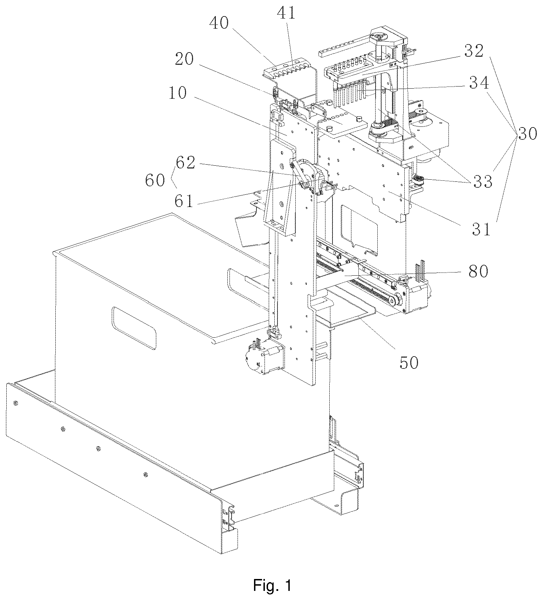

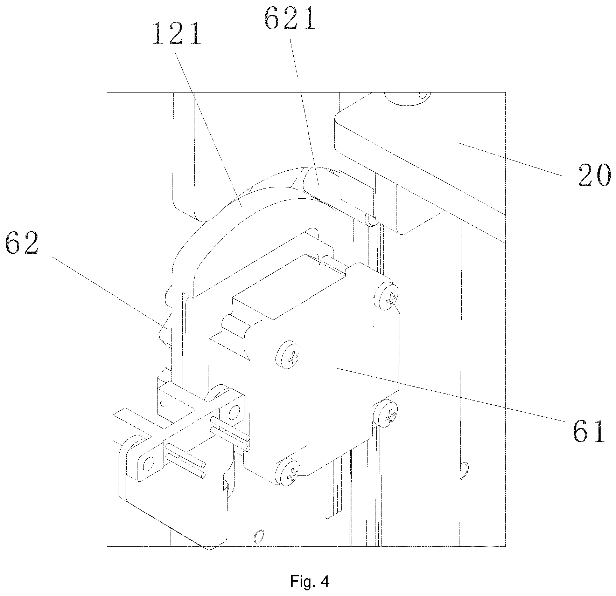

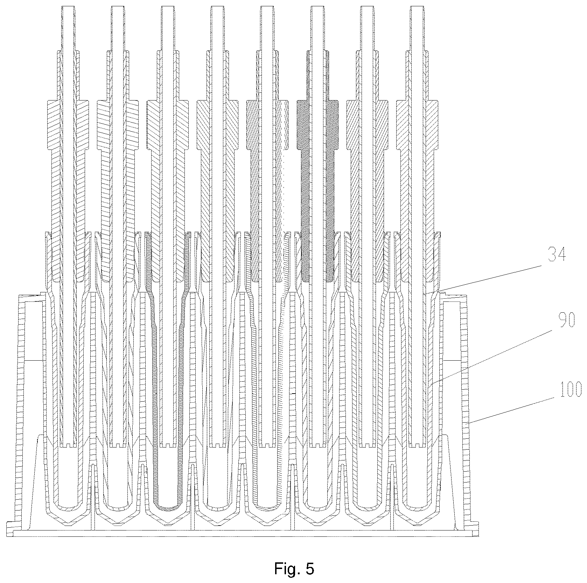

As shown in Figs. 1-5, a consumable recycling apparatus of the embodiment includes: a bearing frame 10, a supporting plate structure 20, a recycling structure 30, and a stripping structure 40. The supporting plate structure 20 is arranged on the bearing frame 10, and the supporting plate structure 20 is provided with a consumable bearing area. The recycling structure 30 is adjacent to the bearing frame 10. The recycling structure 30 includes a rack 31, a mounting beam 32 movably arranged on the rack 31, and a driving structure 33 arranged on the rack 31 and driving the mounting beam 32 to move. The mounting beam 32 is provided with a plurality of recycling rods 34 arranged at intervals in a horizontal direction, and each of the recycling rods 34 is internally provided with an airflow channel. The stripping structure 40 is arranged on the bearing frame 10 and spaced from the supporting plate structure 20. The stripping structure 40 includes a plurality of notch structures 41. The plurality of notch structures 41 are arranged in one-to-one correspondence with the plurality of recycling rods 34. A width of the notch structure 41 is greater than a diameter of the recycling rod 34. The mounting beam 32 has a working position for cooperating with the supporting plate structure 20 and a separating position for cooperating with the stripping structure 40. When the mounting beam 32 is at the working position, the recycling rod 34 is capable of moving close to or away from the supporting plate structure 20. When the mounting beam 32 is at the separating position, each of the recycling rods 34 extends into one of the notch structures 41.

By applying the technical solution of the embodiment, the consumable recycling apparatus includes the bearing frame 10, the bearing frame 10 is provided with the supporting plate structure 20, the supporting plate structure 20 is provided with the consumable bearing area, and a nucleic acid extraction plate 100 may be placed on the consumable bearing area. The consumable recycling apparatus further includes the recycling structure 30 and the stripping structure 40. The recycling structure 30 includes the rack 31, the mounting beam 32 movably arranged on the rack 31, and the driving structure 33 arranged on the rack 31 and driving the mounting beam 32 to move. The mounting beam 32 is provided with the plurality of recycling rods 34 arranged at intervals in an extension direction of the mounting beam 32. The recycling rod 34 may be driven by the driving structure 33 to extend into a magnetic rod sleeve 90, and the magnetic rod sleeve 90 may be mounted on the recycling rod 34. Then the driving structure 33 may move the recycling rod 34 having the magnetic rod sleeve 90 into the notch structure 41 on the stripping structure 40, and enable the magnetic rod sleeve 90 to be located below the stripping structure 40. The width of the notch structure 41 is greater than the diameter of the recycling rod 34, such that the recycling rod 34 may be driven by the driving structure 33 to move upward, the magnetic rod sleeve 90 having a greater diameter may be stopped by the stripping structure 40 and stripped, and the recycling rod 34 may be pulled out from the notch structure 41, so as to achieve the stripping of the magnetic rod sleeve 90. Meanwhile, the recycling rod 34 is further internally provided with the airflow channel, and the airflow channel is in communication with a negative pressure device, such that the recycling rod 34 may further extend into an accommodating hole on the nucleic acid extraction plate 100 so as to suck liquid from the nucleic acid extraction plate 100, and the waste liquid is recycled. By designing the integrated consumable recycling apparatus, the above structure reduces space occupied by the consumable recycling apparatus and improves a space utilization rate of the nucleic acid test analyzer.

It should be noted that the mounting beam 32 may move between the stripping structure 40 and the supporting plate structure 20, such that the mounting beam 32 may be switched between the working position and the separating position.

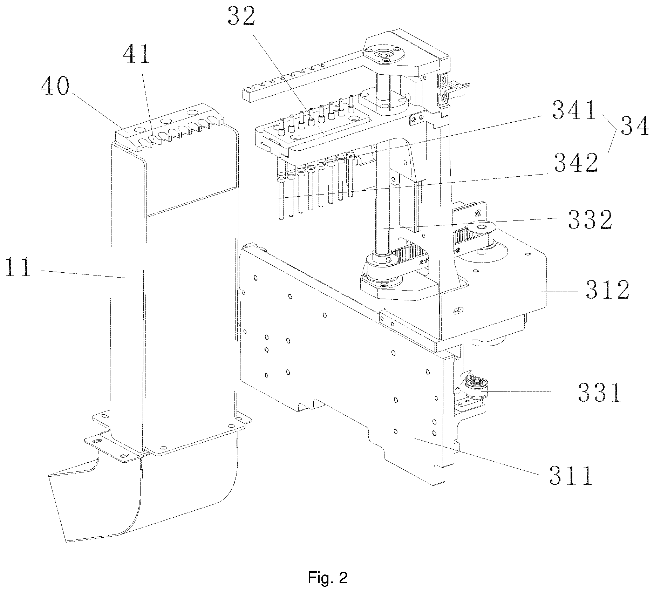

Further, it should be noted that in the embodiment, one of the magnetic rod sleeve 90 and the recycling rod 34 is provided with a groove, and the other one thereof is provided with a protrusion. The recycling rod 34 extends into the magnetic rod sleeve 90 and presses the magnetic rod sleeve 90, the protrusion on the magnetic rod sleeve 90/the recycling rod 34 may be inserted into the groove on the recycling rod 34/the magnetic rod sleeve 90, namely the magnetic rod sleeve 90 may be connected with the recycling rod 34 in a snap-fitted manner. Certainly, in other embodiments, the magnetic rod sleeve 90 and the recycling rod 34 may also be plugged together in an interference manner. Specifically, as shown in Figs. 1 and 2, in the embodiment, the stripping structure 40 and the supporting plate structure 20 are arranged at an interval in a preset direction, an opening of the notch structure 41 faces the supporting plate structure 20, the rack 31 includes a fixed plate 311 and a movable plate 312 movably arranged on the fixed plate 311 in the preset direction, the mounting beam 32 is movably arranged on the movable plate 312 in a vertical direction, the driving structure 33 includes a horizontal driving structure 331 and a vertical driving structure 332, the horizontal driving structure 331 is arranged between the fixed plate 311 and the movable plate 312 so as to drive the movable plate 312 to move, and the vertical driving structure 332 is arranged between the movable plate 312 and the mounting beam 32 so as to drive the mounting beam 32 to move. In the above structure, the horizontal driving structure 331 of the driving structure 33 may drive the mounting beam 32 to move in the preset direction, such that the mounting beam 32 may move between the working position and the separating position. The mounting beam 32 is arranged on the movable plate by the vertical driving structure 332, and the vertical driving structure 332 may drive the mounting beam 32 to move in the vertical direction, such that the recycling rod 34 may extend into and move out of the nucleic acid extraction plate 100, the recycling rod 34 may further be driven to move, and the recycling rod 34 may cooperate with the stripping structure 40. The above structure is simple, driving is stable, and an automation degree of the consumable recycling apparatus is improved advantageously.

As shown in Fig. 2, in the embodiment, the bearing frame 10 includes a guide housing 11 and a vertical plate 12 arranged at a side of the guide housing 11, the guide housing 11 is internally provided with a recycling channel, the stripping structure 40 is arranged at an entrance of the recycling channel, and the supporting plate structure 20 is arranged on the vertical plate 12. In the above structure, the guide housing 11 is internally provided with the recycling channel, and the stripped magnetic rod sleeve 90 may slide down the recycling channel into a collection box, such that centralized recycling is achieved, and subsequent processing of the magnetic rod sleeve 90 is facilitated.

It should be noted that the bearing frame 10 may be of an integrated structure or a split structure. In the embodiment, the vertical plate 12 is separated from the guide housing 11, and the bearing frame 10 is of a split structure. Certainly, in other embodiments, the vertical plate 12 and the guide housing 11 may also be of an integrated structure, that is, the vertical plate 12 and the guide housing 11 are connected to each other.

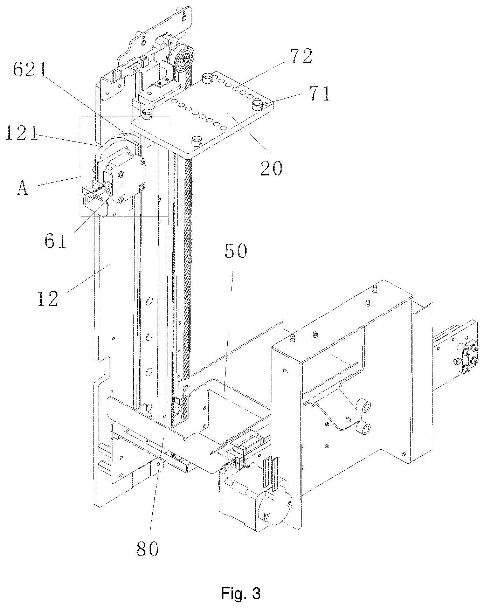

As shown in Figs. 1 and 3, in the embodiment, the supporting plate structure 20 is movably arranged on the bearing frame 10 in the vertical direction, the consumable recycling apparatus further includes a separating frame 50, the separating frame 50 is arranged on the bearing frame 10, and the separating frame 50 is provided with an avoidance hole for the supporting plate structure 20 to pass downward. In the above structure, after the magnetic rod sleeves 90 and the waste liquid in the nucleic acid extraction plate 100 are recycled, the supporting plate structure 20 may be driven to move downward until the supporting plate structure 20 penetrates downward from the avoidance hole of a separating frame, and the nucleic acid extraction plate 100 may be left on the separating frame 50, such that recycling of the nucleic acid extraction plate 100 is facilitated.

As shown in Figs. 1, 3 and 4, in the embodiment, the consumable recycling apparatus further includes a stop structure 60, and the stop structure 60 is movably arranged on the vertical plate 12. The stop structure 60 has a stop position below the supporting plate structure 20 and an avoidance position misaligned with the supporting plate structure 20. When the mounting beam 32 is at the working position, the stop structure 60 is at the stop position so as to support the supporting plate structure 20. In the above structure, when the recycling rod 34 is connected to the magnetic rod sleeve 90, the magnetic rod sleeve 90 needs to be pressed downward, so that the magnetic rod sleeve 90 is fixed to the recycling rod 34 in an interference manner, such that the supporting plate structure 20 needs to bear the pressure of the recycling rod 34. In the invention, the vertical plate 12 is provided with the stop structure 60. When the mounting beam 32 is at the working position, the stop structure 60 is at the stop position so as to support the supporting plate structure 20, such that bearing capability of the supporting plate structure 20 is ensured.

Specifically, as shown in Figs. 1, 3 and 4, in the embodiment, the stop structure 60 includes a driving motor 61 and a swinging member 62 arranged on the vertical plate 12, a first end of the swinging member 62 is in driving connection with a pivot shaft of the driving motor 61, a second end of the swinging member 62 has the stop position and the avoidance position, and the driving motor 61 is capable of driving the second end of the swinging member 62 to move between the stop position and the avoidance position. In the above structure, the driving motor 61 may drive the swinging member 62 to swing. Before the recycling rod 34 is connected to the magnetic rod sleeve 90, the second end of the swinging member 62 is rotated to the stop position, then the supporting plate structure 20 abuts against the second end of the swinging member 62, and then a recycling operation of the magnetic rod sleeve 90 may be started. After the magnetic rod sleeve 90 and the waste liquid in the nucleic acid extraction plate 100 are recycled, the second end of the swinging member 62 may be driven to rotate to the avoidance position. In this case, the supporting plate structure 20 may move downward, the nucleic acid extraction plate 100 may be left on the separating frame 50, so that the nucleic acid extraction plate 100 is recycled. As shown in Figs. 1, 3 and 4, in the embodiment, the driving motor 61 penetrates the vertical plate 12, the vertical plate 12 is further provided with an arc-shaped guide groove 121, and the second end of the swinging member 62 is provided with a supporting rod 621 capable of sliding in the arc-shaped guide groove 121. In the above structure, the arc-shaped guide groove 121 may guide the movement of the second end of the swinging member 62, such that the second end of the swinging member 62 may move stably between the stop position and the avoidance position.

It should be noted that the driving motor 61 and the supporting plate structure 20 are located at a first side of the vertical plate 12, and a rotating shaft of the driving motor 61 penetrates the vertical plate 12 and extends to a second side of the vertical plate 12. The first end of the swinging member 62 is located at the second side of the vertical plate 12 and is in driving connection with the rotating shaft of the driving motor 61. The second end of the swinging member 62 is provided with the supporting rod 621, and the supporting rod 621 penetrates into the first side of the vertical plate 12 through the arc-shaped guide groove 121, so as to achieve stop cooperation with the supporting plate structure 20.

As shown in Figs. 1 and 3, in the embodiment, the consumable recycling apparatus further includes a pushing structure 80. The pushing structure 80 includes a mounting seat arranged on the bearing frame 10 and a push rod movably arranged on the mounting seat. The push rod is capable of pushing a consumable to leave the separating frame 50. In the above structure, the pushing structure may push the nucleic acid extraction plate 100 on the separating frame 50 to leave the separating frame 50. On one hand, recycling of the nucleic acid extraction plate 100 is facilitated. On the other hand, a position on the separating frame 50 may be left empty, and recycling of the subsequent nucleic acid extraction plate 100 is facilitated.

As shown in Fig. 3, in the embodiment, the supporting plate structure 20 is provided with a limiting structure configured to limit the consumable. In the above structure, the limiting structure may increase placement stability of the nucleic acid extraction plate 100 on the supporting plate structure 20.

As shown in Fig. 3, in the embodiment, the limiting structure includes a plurality of limiting columns 71 arranged on the supporting plate structure 20. The plurality of limiting columns 71 may cooperate with insertion holes at a bottom of the nucleic acid extraction plate 100 in an inserted manner, such that the nucleic acid extraction plate 100 may be stably fixed on the supporting plate structure 20.

As shown in Fig. 3, in the embodiment, the limiting structure further includes a magnetic attraction structure 72 arranged on the supporting plate structure 20. In the above structure, the magnetic attraction structure 72 may cooperate with magnetic beads in the nucleic acid extraction plate 100 in a magnetic attraction manner, such that the magnetic beads may be adsorbed on the bottom of the nucleic acid extraction plate 100, and the situation that the airflow channel of the recycling rod 34 is blocked due to movement of the magnetic beads when the recycling rod 34 sucks the waste liquid in the nucleic acid extraction plate 100 is avoided.

As shown in Figs. 1 and 2, in the embodiment, the recycling rod 34 includes a first rod section 341 connected to the mounting beam 32 and a second rod section 342 located below the first rod section 341, a diameter of the first rod section 341 is greater than a diameter of the second rod section 342, the first rod section 341 is provided with a diameter-changing portion having a diameter gradually decreasing from top to bottom, and the airflow channel penetrates the first rod section 341 and the second rod section 342. In the above structure, the recycling rod 34 needs to cooperate with the magnetic rod sleeve 90, such that setting the first rod section 341 to have a greater diameter and arrangement of the diameter-changing portion on the first rod section 341 may facilitate insertion of the recycling rod 34 into the magnetic rod sleeve 90. Setting the second rod section 342 to have a smaller diameter may enable the second rod section 342 to smoothly extend into the nucleic acid extraction plate 100.

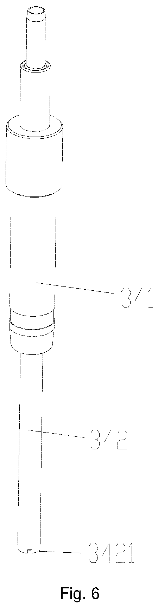

As shown in Fig. 6, it should also be noted that a bottom of the second rod section 342 is provided with a plurality of notches 3421, such that when the bottom of the second rod section 342 is in contact with an inner wall of the nucleic acid extraction plate 100, the airflow channel will not be blocked, so that the waste liquid in the nucleic acid extraction plate 100 may be ensured to be completely sucked.

The invention further provides a nucleic acid test analyzer. An embodiment of the nucleic acid test analyzer includes a consumable recycling apparatus. The consumable recycling apparatus is the consumable recycling apparatus described above. In the above structure, the consumable recycling apparatus has advantages of a high integration level and small occupied space, so the nucleic acid test analyzer having the consumable recycling apparatus also has the above advantages.

In the description of the present invention, it should be understood that orientations or positional relations indicated by the orientation terms "front, rear, upper, lower, left and right", "transverse, vertical, perpendicular, and horizontal", "top and bottom", etc. are based on the orientations or positional relations shown in the accompanying drawings and are only for facilitating the description of the present invention and simplifying the description, and in the absence of a statement to the contrary, the orientation terms do not indicate or imply that an apparatus or element referred to must have a particular orientation or be constructed and operated in a particular orientation, and therefore cannot be interpreted as limiting the protection scope of the present invention; and the orientation terms "inside and outside" refer to inside and outside relative to an outline of each component itself.

For convenience of description, spatial relative terms such as "over", "above", "on an upper surface" and "on" can be used herein to describe spatial positional relations of one device or feature with other devices or features as shown in the drawings. It should be understood that the spatial relative terms are intended to include different orientations in use or operation in addition to the orientation of the device described in the drawings. For instance, if the device in the drawings is inverted, the device described as "above" or "over" other devices or structures would then be positioned "below" or "under" the other devices or structures. Therefore, the illustrative term "above" can include two orientations of "above" and "below." The device can also be positioned (rotated 90 degrees or at other orientations) in other different ways and the spatial relative description used herein is interpreted accordingly.

In addition, it should be noted that words "first" and "second" are used to define parts only for convenience of distinguishing corresponding parts. Unless otherwise stated, the above words have no special meaning, so they cannot be understood as limiting the protection scope of the present invention.

The above descriptions are merely preferred embodiments of the present invention and are not intended to limit the present invention, and can be modified and changed by those skilled in the art.

## Claims

1. A consumable recycling apparatus, comprising:
a bearing frame (10);
a supporting plate structure (20) arranged on the bearing frame (10), wherein the supporting plate structure (20) is provided with a consumable bearing area, the consumable bearing area is used for placing a nucleic acid extraction plate (100), and the nucleic acid extraction plate (100) is provided with a magnetic rod sleeve (90) and waste liquid;
a recycling structure (30) adjacent to the bearing frame (10), wherein the recycling structure (30) comprises a rack (31), a mounting beam (32) movably arranged on the rack (31), and a driving structure (33) arranged on the rack (31) and driving the mounting beam (32) to move, the mounting beam (32) is provided with a plurality of recycling rods (34) arranged at intervals in a horizontal direction, and each of the recycling rods (34) is internally provided with an airflow channel, the airflow channel is in communication with a negative pressure device and the recycling rod (34) is capable of extending into an accommodating hole on the nucleic acid extraction plate (100) so as to suck the waste liquid from the nucleic acid extraction plate (100); and
a stripping structure (40) arranged on the bearing frame (10), wherein the stripping structure (40) comprises a plurality of notch structures (41), the plurality of notch structures (41) are arranged in one-to-one correspondence with the plurality of recycling rods (34), and a width of the notch structure (41) is greater than a diameter of the recycling rod (34),
wherein the mounting beam (32) has a working position for cooperating with the supporting plate structure (20) and a separating position for cooperating with the stripping structure (40), the mounting beam (32) is capable of moving between the stripping structure (40) and the supporting plate structure (20), such that the mounting beam (32) can be switched between the working position and the separating position, when a nucleic acid extraction plate (100) provided with a magnetic rod sleeve (90) and waste liquid is placed on the consumable bearing area and when the mounting beam (32) is at the working position, the recycling rod (34) is capable of moving close to or away from the supporting plate structure (20) so as to suck the waste liquid from the nucleic acid extraction plate (100) and mount the magnetic rod sleeve (90), and when the mounting beam (32) is at the separating position, each of the recycling rods (34) extends into one of the notch structures (41) and enables the magnetic rod sleeve (90) to be located below the stripping structure (40).

2. The consumable recycling apparatus according to claim 1, wherein the stripping structure (40) and the supporting plate structure (20) are arranged at an interval in a preset direction, an opening of the notch structure (41) faces the supporting plate structure (20), the rack (31) comprises a fixed plate (311) and a movable plate (312) movably arranged on the fixed plate (311) in the preset direction , the mounting beam (32) is movably arranged on the movable plate (312) in a vertical direction, the driving structure (33) comprises a horizontal driving structure (331) and a vertical driving structure (332), the horizontal driving structure (331) is arranged between the fixed plate (311) and the movable plate (312) so as to drive the movable plate (312) to move, and the vertical driving structure (332) is arranged between the movable plate (312) and the mounting beam (32) so as to drive the mounting beam (32) to move.

3. The consumable recycling apparatus according to claim 2, wherein the bearing frame (10) comprises a guide housing (11) and a vertical plate (12) arranged at a side of the guide housing (11), the guide housing (11) is internally provided with a recycling channel, the stripping structure (40) is arranged at an entrance of the recycling channel, and the supporting plate structure (20) is arranged on the vertical plate (12).

4. The consumable recycling apparatus according to claim 3, wherein the supporting plate structure (20) is movably arranged on the bearing frame (10) in the vertical direction, and the consumable recycling apparatus further comprises:
a separating frame (50) arranged on the bearing frame (10), wherein the separating frame (50) is provided with an avoidance hole for the supporting plate structure (20) to pass downward.

5. The consumable recycling apparatus according to claim 3, further comprising:
a stop structure (60) movably arranged on the vertical plate (12), wherein the stop structure (60) has a stop position below the supporting plate structure (20) and an avoidance position misaligned with the supporting plate structure (20), and when the mounting beam (32) is at the working position, the stop structure (60) is at the stop position so as to support the supporting plate structure (20).

6. The consumable recycling apparatus according to claim 5, wherein the stop structure (60) comprises a driving motor (61) and a swinging member (62) arranged on the vertical plate (12), a first end of the swinging member (62) is in driving connection with a pivot shaft of the driving motor (61), a second end of the swinging member (62) has the stop position and the avoidance position, and the driving motor (61) is capable of driving the second end of the swinging member (62) to move between the stop position and the avoidance position.

7. The consumable recycling apparatus according to claim 6, wherein the driving motor (61) penetrates the vertical plate (12), the vertical plate (12) is further provided with an arc-shaped guide groove (121), and the second end of the swinging member (62) is provided with a supporting rod (621) capable of sliding in the arc-shaped guide groove (121).

8. The consumable recycling apparatus according to claim 4, further comprising:
a pushing structure (80) comprising a mounting seat arranged on the bearing frame (10) and a push rod movably arranged on the mounting seat, wherein the push rod is capable of pushing a consumable to leave the separating frame (50).

9. The consumable recycling apparatus according to claim 1, wherein the supporting plate structure (20) is provided with a limiting structure configured to limit a consumable.

10. The consumable recycling apparatus according to claim 9, wherein
the limiting structure comprises a plurality of limiting columns (71) arranged on the supporting plate structure (20); and/or
the limiting structure comprises a magnetic attraction structure (72) arranged on the supporting plate structure (20).

11. The consumable recycling apparatus according to claim 1, wherein the recycling rod (34) comprises a first rod section (341) connected to the mounting beam (32) and a second rod section (342) located below the first rod section (341), a diameter of the first rod section (341) is greater than a diameter of the second rod section (342), the first rod section (341) is provided with a diameter-changing portion having a diameter gradually decreasing from top to bottom, and the airflow channel penetrates the first rod section (341) and the second rod section (342).

12. The consumable recycling apparatus according to claim 1, wherein the recycling rod (34) is configured to connect a magnetic rod sleeve (90), one of the magnetic rod sleeve (90) and the recycling rod (34) is provided with a groove, and the other one of the magnetic rod sleeve (90) and the recycling rod (34) is provided with a protrusion, and the recycling rod (34) extends into the magnetic rod sleeve (90) and presses the magnetic rod sleeve (90), and the magnetic rod sleeve (90) is connected with the recycling rod (34) in a snap-fitted manner.

13. The consumable recycling apparatus according to claim 7, wherein the driving motor (61) and the supporting plate structure (20) are located at a first side of the vertical plate (12), and the pivot shaft of the driving motor (61) penetrates the vertical plate (12) and extends to a second side of the vertical plate (12), the first end of the swinging member (62) is located at the second side of the vertical plate (12), the supporting rod (621) penetrates into the first side of the vertical plate (12) through the arc-shaped guide groove (121), when the second end of the swinging member (62) is rotated to the stop position, the supporting rod (321) being in stop cooperation with the supporting plate structure (20), when the second end of the swinging member (62) is rotated to the avoidance position, the supporting plate structure (20) is able to move downward.

14. The consumable recycling apparatus according to claim 11, a bottom of the second rod section (342) is provided with a plurality of notches (3421).

15. A nucleic acid test analyzer, comprising:
a consumable recycling apparatus, wherein the consumable recycling apparatus is the consumable recycling apparatus according to any one of claims 1-14.

## Patentansprüche

1. Vorrichtung zur Wiederaufbereitung von Verbrauchsmaterial, umfassend:
einen Tragrahmen (10);
eine Trägerplattenstruktur (20), die auf dem Tragrahmen (10) angeordnet ist, wobei die Trägerplattenstruktur (20) mit einem Verbrauchsmaterial-Lagerbereich versehen ist, der Verbrauchsmaterial-Lagerbereich zum Platzieren einer Nukleinsäure-Extraktionsplatte (100) verwendet wird und die Nukleinsäure-Extraktionsplatte (100) mit einer Magnetstabhülse (90) und einer Abfallflüssigkeit versehen ist;
eine Wiederaufbereitungsstruktur (30), die an den Tragrahmen (10) angrenzt, wobei die Wiederaufbereitungsstruktur (30) ein Gestell (31), einen Montageträger (32), der beweglich auf dem Gestell (31) angeordnet ist, und eine Antriebsstruktur (33) umfasst, die auf dem Gestell (31) angeordnet ist und den Montageträger (32) zur Bewegung antreibt, wobei der Montageträger (32) mit einer Vielzahl von Wiederaufbereitungsstäben (34) versehen ist, die in Abständen in einer horizontalen Richtung angeordnet sind, und jeder der Wiederaufbereitungsstäbe (34) innen mit einem Luftströmungskanal versehen ist, wobei der Luftströmungskanal mit einer Unterdruckvorrichtung in Verbindung steht und der Wiederaufbereitungsstab (34) in der Lage ist, sich in ein Aufnahmeloch auf der Nukleinsäure-Extraktionsplatte (100) zu erstrecken, um die Abfallflüssigkeit von der Nukleinsäure-Extraktionsplatte (100) abzusaugen; und
eine Abstreifstruktur (40), die auf dem Tragrahmen (10) angeordnet ist, wobei die Abstreifstruktur (40) eine Vielzahl von Kerbstrukturen (41) umfasst, wobei die Vielzahl von Kerbstrukturen (41) in eins-zu-eins-Entsprechung mit der Vielzahl von Wiederaufbereitungsstäben (34) angeordnet ist und wobei eine Breite der Kerbstruktur (41) größer als ein Durchmesser des Wiederaufbereitungsstabs (34) ist, wobei der Montageträger (32) eine Arbeitsposition zum Zusammenwirken mit der Trägerplattenstruktur (20) und eine Trennposition zum Zusammenwirken mit der Abstreifstruktur (40) aufweist, wobei der Montageträger (32) in der Lage ist, sich zwischen der Abstreifstruktur (40) und der Trägerplattenstruktur (20) zu bewegen, so dass der Montageträger (32) zwischen der Arbeitsposition und der Trennposition umgeschaltet werden kann, wenn eine Nukleinsäure-Extraktionsplatte (100), die mit einer Magnetstabhülse (90) und einer Abfallflüssigkeit versehen ist, auf der Verbrauchsmaterial-Lagerfläche platziert wird und wenn der Montageträger (32) sich in der Arbeitsposition befindet, ist der Wiederaufbereitungsstab (34) in der Lage, sich nahe an die Trägerplattenstruktur (20) heranzubewegen oder von ihr wegzubewegen, um die Abfallflüssigkeit von der Nukleinsäure-Extraktionsplatte (100) abzusaugen und die Magnetstabhülse (90) anzubringen, und wenn sich der Montageträger (32) in der Trennposition befindet, erstreckt sich jeder der Wiederaufbereitungsstäbe (34) in eine der Kerbstrukturen (41) und ermöglicht, dass die Magnetstabhülse (90) unter der Abstreifstruktur (40) angeordnet wird.

2. Vorrichtung zur Wiederaufbereitung von Verbrauchsmaterial nach Anspruch 1, wobei die Abstreifstruktur (40) und die Trägerplattenstruktur (20) in einem Abstand in einer vorgegebenen Richtung angeordnet sind, eine Öffnung der Kerbstruktur (41) der Trägerplattenstruktur (20) zugewandt ist, das Gestell (31) eine feste Platte (311) und eine bewegliche Platte (312) umfasst, die in der vorgegebenen Richtung beweglich auf der festen Platte (311) angeordnet ist, der Montageträger (32) in einer vertikalen Richtung beweglich auf der beweglichen Platte (312) angeordnet ist, die Antriebsstruktur (33) eine horizontale Antriebsstruktur (331) und eine vertikale Antriebsstruktur (332) umfasst, wobei die horizontale Antriebsstruktur (331) zwischen der festen Platte (311) und der beweglichen Platte (312) angeordnet ist, um die bewegliche Platte (312) zu bewegen, und die vertikale Antriebsstruktur (332) zwischen der beweglichen Platte (312) und dem Montageträger (32) angeordnet ist, um den Montageträger (32) zu bewegen.

3. Vorrichtung zur Wiederaufbereitung von Verbrauchsmaterial nach Anspruch 2, wobei der Tragrahmen (10) ein Führungsgehäuse (11) und eine vertikale Platte (12) umfasst, die an einer Seite des Führungsgehäuses (11) angeordnet ist, das Führungsgehäuse (11) innen mit einem Wiederaufbereitungskanal versehen ist, die Abstreifstruktur (40) an einem Eingang des Wiederaufbereitungskanals angeordnet ist und die Trägerplattenstruktur (20) an der vertikalen Platte (12) angeordnet ist.

4. Vorrichtung zur Wiederaufbereitung von Verbrauchsmaterial nach Anspruch 3, wobei die Trägerplattenstruktur (20) in vertikaler Richtung beweglich auf dem Tragrahmen (10) angeordnet ist und die Vorrichtung zur Wiederaufbereitung von Verbrauchsmaterial ferner umfasst:
einen Trennrahmen (50), der auf dem Tragrahmen (10) angeordnet ist, wobei der Trennrahmen (50) mit einem Ausweichloch versehen ist, durch das die Trägerplattenstruktur (20) nach unten geführt werden kann.

5. Vorrichtung zur Wiederaufbereitung von Verbrauchsmaterial nach Anspruch 3, ferner umfassend:
eine Anschlagstruktur (60), die beweglich auf der vertikalen Platte (12) angeordnet ist, wobei die Anschlagstruktur (60) eine Anschlagposition unterhalb der Trägerplattenstruktur (20) und eine mit der Trägerplattenstruktur (20) fehlausgerichtete Ausweichposition aufweist, und wenn sich der Montageträger (32) in der Arbeitsposition befindet, ist die Anschlagstruktur (60) in der Anschlagposition, um die Trägerplattenstruktur (20) zu stützen.

6. Vorrichtung zur Wiederaufbereitung von Verbrauchsmaterial nach Anspruch 5, wobei die Anschlagstruktur (60) einen Antriebsmotor (61) und ein Schwingelement (62) umfasst, das an der vertikalen Platte (12) angeordnet ist, ein erstes Ende des Schwingelements (62) in Antriebsverbindung mit einer Schwenkwelle des Antriebsmotors (61) steht, ein zweites Ende des Schwingelements (62) die Anschlagposition und die Ausweichposition aufweist und der Antriebsmotor (61) in der Lage ist, das zweite Ende des Schwingelements (62) anzutreiben, um es zwischen der Anschlagposition und der Ausweichposition zu bewegen.

7. Vorrichtung zur Wiederaufbereitung von Verbrauchsmaterial nach Anspruch 6, wobei der Antriebsmotor (61) in die vertikale Platte (12) eindringt, die vertikale Platte (12) ferner mit einer bogenförmigen Führungsnut (121) versehen ist und das zweite Ende des Schwingelements (62) mit einer Stützstange (621) versehen ist, die in der bogenförmigen Führungsnut (121) gleiten kann.

8. Vorrichtung zur Wiederaufbereitung von Verbrauchsmaterial nach Anspruch 4, ferner umfassend:
eine Schiebestruktur (80), die einen auf dem Tragrahmen (10) angeordneten Montagesitz und eine beweglich auf dem Montagesitz angeordnete Schiebestange umfasst, wobei die Schiebestange in der Lage ist, ein Verbrauchsmaterial zu schieben, um den Trennrahmen (50) zu verlassen.

9. Vorrichtung zur Wiederaufbereitung von Verbrauchsmaterial nach Anspruch 1, wobei die Trägerplattenstruktur (20) mit einer Begrenzungsstruktur versehen ist, die zur Begrenzung eines Verbrauchsmaterials konfiguriert ist.

10. Vorrichtung zur Wiederaufbereitung von Verbrauchsmaterial nach Anspruch 9, wobei die Begrenzungsstruktur eine Vielzahl von Begrenzungssäulen (71) umfasst, die auf der Trägerplattenstruktur (20) angeordnet sind; und/oder
die Begrenzungsstruktur eine magnetische Anziehungsstruktur (72) umfasst, die auf der Trägerplattenstruktur (20) angeordnet ist.

11. Vorrichtung zur Wiederaufbereitung von Verbrauchsmaterial nach Anspruch 1, wobei der Wiederaufbereitungsstab (34) einen ersten Stababschnitt (341), der mit dem Montageträger (32) verbunden ist, und einen zweiten Stababschnitt (342) umfasst, der unterhalb des ersten Stababschnitts (341) angeordnet ist, ein Durchmesser des ersten Stababschnitts (341) größer ist als ein Durchmesser des zweiten Stababschnitts (342), der erste Stababschnitt (341) mit einem durchmesserverändernden Abschnitt versehen ist, der einen von oben nach unten allmählich abnehmenden Durchmesser aufweist, und der Luftströmungskanal in den ersten Stababschnitt (341) und in den zweiten Stababschnitt (342) eindringt.

12. Vorrichtung zur Wiederaufbereitung von Verbrauchsmaterial nach Anspruch 1, wobei der Wiederaufbereitungsstab (34) so konfiguriert ist, dass er mit einer Magnetstabhülse (90) verbunden ist, eines aus der Magnetstabhülse (90) und dem Wiederaufbereitungsstab (34) mit einer Nut versehen ist und das andere aus der Magnetstabhülse (90) und dem Wiederaufbereitungsstab (34) mit einem Vorsprung versehen ist, und der Wiederaufbereitungsstab (34) sich in die Magnetstabhülse (90) erstreckt und die Magnetstabhülse (90) drückt, und die Magnetstabhülse (90) mit dem Wiederaufbereitungsstab (34) in einer Schnappverbindung verbunden ist.

13. Vorrichtung zur Wiederaufbereitung von Verbrauchsmaterial nach Anspruch 7, wobei der Antriebsmotor (61) und die Trägerplattenstruktur (20) an einer ersten Seite der vertikalen Platte (12) angeordnet sind und die Schwenkwelle des Antriebsmotors (61) in die vertikale Platte (12) eindringt und sich zu einer zweiten Seite der vertikalen Platte (12) erstreckt, wobei das erste Ende des Schwingelements (62) an der zweiten Seite der vertikalen Platte (12) angeordnet ist, die Stützstange (621) in die erste Seite der vertikalen Platte (12) durch die bogenförmige Führungsnut (121) eindringt, wenn das zweite Ende des Schwingelements (62) in die Anschlagposition gedreht wird, wobei die Stützstange (321) in Anschlagzusammenwirkung mit der Trägerplattenstruktur (20) ist, wenn das zweite Ende des Schwingelements (62) in die Ausweichposition gedreht wird, kann sich die Trägerplattenstruktur (20) nach unten bewegen.

14. Vorrichtung zur Wiederaufbereitung von Verbrauchsmaterial nach Anspruch 11, wobei ein Boden des zweiten Stababschnitts (342) mit einer Vielzahl von Kerben (3421) versehen ist.

15. Nukleinsäuretest-Analysator, umfassend:
eine Vorrichtung zur Wiederaufbereitung von Verbrauchsmaterial, wobei die Vorrichtung zur Wiederaufbereitung von Verbrauchsmaterial die Vorrichtung zur Wiederaufbereitung von Verbrauchsmaterial nach einem der Ansprüche 1 bis 14 ist.

## Revendications

1. Appareil de recyclage de produits consommables comprenant :
un cadre porteur (10) ;
une structure de plaque de support (20) disposée sur le cadre porteur (10), la structure de plaque de support (20) étant pourvue d'une surface d'appui de produits consommables, la surface d'appui de produits consommables étant utilisée pour placer une plaque d'extraction (100) d'acide nucléique, et la plaque d'extraction (100) d'acide nucléique est pourvue d'un manchon de tige magnétique (90) et d'un liquide résiduaire ;
une structure de recyclage (30) adjacente au cadre porteur (10), la structure de recyclage (30) comprenant une crémaillère (31), une barre de montage (32) disposée de manière mobile sur la crémaillère (31), et une structure d'entraînement (33) disposée sur la crémaillère (31) et entraînant le déplacement de la barre de montage (32), la barre de montage (32) étant pourvue d'une pluralité de tiges de recyclage (34) disposées à des intervalles réguliers dans une direction horizontale, et chacune des tiges de recyclage (34) étant pourvue intérieurement d'un canal d'écoulement d'air, le canal d'écoulement d'air étant en communication avec un dispositif de pression négative et la tige de recyclage (34) étant capable de s'étendre dans un trou de logement sur la plaque d'extraction (100) d'acide nucléique de manière à aspirer le liquide résiduaire de la plaque d'extraction (100) d'acide nucléique ; et
une structure de démontage (40) disposée sur le cadre porteur (10), la structure de démontage (40) comprenant une pluralité de structures à encoches (41), la pluralité de structures à encoches (41) étant disposées en correspondance une à une avec la pluralité de tiges de recyclage (34), et une largeur de la structure à encoches (41) est supérieure à un diamètre de la tige de recyclage (34),
la barre de montage (32) présentant une position de travail pour coopérer avec la structure de plaque de support (20) et une position de séparation pour coopérer avec la structure de démontage (40), la barre de montage (32) étant capable de se déplacer entre la structure de démontage (40) et la structure de plaque de support (20), de sorte que la barre de montage (32) puisse passer de la position de travail à la position de séparation, lorsqu'une plaque d'extraction (100) d'acide nucléique pourvue d'un manchon de tige magnétique (90) et d'un liquide résiduaire est placée sur la surface d'appui des produits consommables et que la barre de montage (32) se trouve en position de travail, la tige de recyclage (34) pouvant se rapprocher ou s'éloigner de la structure de plaque de support (20) afin d'aspirer le liquide résiduaire de la plaque d'extraction (100) d'acide nucléique et de monter le manchon de tige magnétique (90), et lorsque la barre de montage (32) se trouve en position de séparation, chacune des tiges de recyclage (34) s'étend dans l'une des structures à encoche (41) et permet au manchon de tige magnétique (90) d'être situé sous la structure de démontage (40).

2. Appareil de recyclage de produits consommables selon la revendication 1, la structure de démontage (40) et la structure de plaque de support (20) étant disposées à un intervalle régulier dans une direction prédéfinie, une ouverture de la structure à encoches (41) faisant face à la structure de plaque de support (20), la crémaillère (31) comprenant une plaque fixe (311) et une plaque mobile (312) disposée de manière mobile sur la plaque fixe (311) dans la direction prédéfinie, la barre de montage (32) étant disposée de manière mobile sur la plaque mobile (312) dans une direction verticale, la structure d'entraînement (33) comprenant une structure d'entraînement horizontale (331) et une structure d'entraînement verticale (332), la structure d'entraînement horizontale (331) étant disposée entre la plaque fixe (311) et la plaque mobile (312) de manière à entraîner le déplacement de la plaque mobile (312), et la structure d'entraînement verticale (332) étant disposée entre la plaque mobile (312) et la barre de montage (32) de manière à entraîner le déplacement de la barre de montage (32).

3. Appareil de recyclage de produits consommables selon la revendication 2, le cadre porteur (10) comprenant un logement de guidage (11) et une plaque verticale (12) disposée sur un côté du logement de guidage (11), le logement de guidage (11) étant pourvu intérieurement d'un canal de recyclage, la structure de démontage (40) étant disposée à une entrée du canal de recyclage, et la structure de plaque de support (20) étant disposée sur la plaque verticale (12).

4. Appareil de recyclage de produits consommables selon la revendication 3, la structure de plaque de support (20) étant disposée de manière mobile sur le cadre porteur (10) dans la direction verticale, et l'appareil de recyclage de produits consommables comprenant en outre :
un cadre de séparation (50) disposé sur le cadre porteur (10), le cadre de séparation (50) étant pourvu d'un trou d'évitement permettant à la structure de plaque de support (20) de passer vers le bas.

5. Appareil de recyclage de produits consommables selon la revendication 3, comprenant en outre :
une structure d'arrêt (60) disposée de manière mobile sur la plaque verticale (12), la structure d'arrêt (60) ayant une position d'arrêt en dessous de la structure de plaque de support (20) et une position d'évitement désalignée par rapport à la structure de plaque de support (20), et lorsque la barre de montage (32) se trouve en position de travail, la structure d'arrêt (60) se trouve en position d'arrêt de manière à soutenir la structure de plaque de support (20).

6. Appareil de recyclage de produits consommables selon la revendication 5, la structure d'arrêt (60) comprenant un moteur d'entraînement (61) et un élément oscillant (62) disposés sur la plaque verticale (12), une première extrémité de l'élément oscillant (62) étant en liaison d'entraînement avec un axe de pivot du moteur d'entraînement (61), une seconde extrémité de l'élément oscillant (62) ayant la position d'arrêt et la position d'évitement, et le moteur d'entraînement (61) étant capable d'entraîner la seconde extrémité de l'élément oscillant (62) pour se déplacer entre la position d'arrêt et la position d'évitement.

7. Appareil de recyclage de produits consommables selon la revendication 6, le moteur d'entraînement (61) pénétrant la plaque verticale (12), la plaque verticale (12) étant en outre pourvue d'une rainure de guidage arquée (121), et la seconde extrémité de l'élément oscillant (62) étant pourvue d'une tige de support (621) capable de coulisser dans la rainure de guidage arquée (121).

8. Appareil de recyclage de produits consommables selon la revendication 4, comprenant en outre :
une structure de poussée (80) comprenant un siège de montage disposé sur le cadre porteur (10) et une tige de poussée disposée de manière mobile sur le siège de montage, la tige de poussée étant capable de pousser un produit consommable pour qu'il quitte le cadre de séparation (50).

9. Appareil de recyclage de produits consommables selon la revendication 1, la structure de plaque de support (20) étant pourvue d'une structure de limitation configurée pour limiter un consommable.

10. Appareil de recyclage de produits consommables selon la revendication 9, la structure de limitation comprenant plusieurs colonnes de limitation (71) disposées sur la structure de plaque de support (20) ; et/ou
la structure de limitation comprenant une structure d'attraction magnétique (72) disposée sur la structure de plaque de support (20).

11. Appareil de recyclage de produits consommables selon la revendication 1, la tige de recyclage (34) comprenant une première section de tige (341) reliée à la barre de montage (32) et une seconde section de tige (342) située sous la première section de tige (341), le diamètre de la première section de tige (341) étant supérieur au diamètre de la seconde section de tige (342), la première section de tige (341) étant pourvue d'une partie à diamètre variable dont le diamètre diminue progressivement de haut en bas, et le canal d'écoulement d'air pénétrant dans la première section de tige (341) et dans la seconde section de tige (342).

12. Appareil de recyclage de produits consommables selon la revendication 1, la tige de recyclage (34) étant configurée pour relier un manchon de tige magnétique (90), l'un parmi le manchon de tige magnétique (90) et la tige de recyclage (34) étant pourvu d'une rainure, et l'autre parmi le manchon de tige magnétique (90) et la tige de recyclage (34) étant pourvu d'une protubérance, et la tige de recyclage (34) s'étendant dans le manchon de tige magnétique (90) et pressant le manchon de tige magnétique (90), et le manchon de tige magnétique (90) étant relié à la tige de recyclage (34) d'une manière encliquetable.

13. Appareil de recyclage de produits consommables selon la revendication 7, le moteur d'entraînement (61) et la structure de plaque de support (20) étant situés sur un premier côté de la plaque verticale (12), et l'axe de pivot du moteur d'entraînement (61) pénétrant la plaque verticale (12) et s'étendant jusqu'à un second côté de la plaque verticale (12), la première extrémité de l'élément oscillant (62) étant située sur le second côté de la plaque verticale (12), la tige de support (621) pénétrant dans le premier côté de la plaque verticale (12) à travers la rainure de guidage arquée (121), lorsque la seconde extrémité de l'élément oscillant (62) est tournée en position d'arrêt, la tige de support (321) étant en coopération d'arrêt avec la structure de plaque de support (20), lorsque la seconde extrémité de l'élément oscillant (62) est tournée en position d'évitement, la structure de plaque de support (20) étant capable de se déplacer vers le bas.

14. Appareil de recyclage de produits consommables selon la revendication 11, un fond de la seconde section de tige (342) étant pourvu d'une pluralité d'encoches (3421).

15. Analyseur d'essai d'acide nucléique, comprenant :
un appareil de recyclage de produits consommables, l'appareil de recyclage de produits consommables étant l'appareil de recyclage de produits consommables selon l'une quelconque des revendications 1-14.
